# EUROPEAN PATENT APPLICATION

(11) **EP 4 385 975 A1**
(43) Date of publication of application: **19.06.2024**
(21) Application number: 22213902.4
(22) Date of filing: 15.12.2022
(51) Int. Cl.: C07C 407/00, C07C 409/16

(54) **PROCESS FOR PREPARING A TERTIARY ALKYL ORGANIC PEROXIDE**

(71) Applicant: Nouryon Chemicals International B.V., 1101 BZ Amsterdam (NL)
(72) Inventor: VAN DEN BERG, Michel, 1101 BZ Amsterdam (NL); KROSENDIJK, Edgard Everick, 1101 BZ Amsterdam (NL); STEENSMA, Maria, 1101 BZ Amsterdam (NL)
(74) Representative: LKGlobal UK Ltd.

(57) **Abstract**

The present disclosure relates to a process for preparing a tertiary alkyl organic peroxides comprising:
a) in a first condensation stage, reacting a component containing at least one tertiary alcohol group with a compound containing at least one tertiary hydroperoxide function, in the presence of a catalyst and optional co-catalyst, to form a mixture comprising an organic phase comprising the tertiary alkyl organic peroxide and an aqueous phase,
b) in a dewatering stage, separating the aqueous phase from the organic phase, and
c) in a second condensation stage, continuing reacting the component containing at least one tertiary alcohol group with the compound containing at least one tertiary hydroperoxide group, in the presence of a catalyst and optional co-catalyst,

wherein, in the first condensation stage, the mole ratio between the compound containing at least one tertiary hydroperoxide function and the component containing at least one tertiary alcohol group is at least 3:1, and
wherein no dewatering takes place during the first condensation stage.

## Description

### Technical Field

The present disclosure relates to a process for preparing a tertiary alkyl organic peroxide which generates a lower amount of salt waste in the wastewater stream.

### Background

Tertiary alkyl organic peroxides are usually produced by a condensation reaction between a tertiary alcohol and a tertiary hydroperoxide. That reaction generates a substantial amount of water that needs to be removed during the reaction. As such, the reaction is usually performed in the presence of a substantial amount of sulfuric acid (H₂SO₄) and/or with contemporaneous removal of water by azeotropic distillation.

There are several technical challenges with such processes. The requirement of a large amount of sulfuric acid generates a lot of salt waste in the wastewater, which is undesirable (particularly from an environmental viewpoint). Removing water azeotropically during the course of the reaction can be dangerous, because accidentally removing too much water can lead to a runaway reaction, which is particularly unsafe when peroxides are involved. Additionally, the reaction typically needs to be performed at lower temperatures (e.g., at or below about 60°C) to avoid peroxide decomposition under those process conditions. Peroxide decomposition would lower the yield and introduce further safety issues. The lower temperature results in slower reaction and thus longer reaction times, which is unfavourable from an industrial process viewpoint.

US5312998 discloses a process for preparing ditertiary butyl peroxide by a condensation reaction between a substantial excess of t-butyl alcohol with t-butyl hydroperoxide in the presence of phosphotungstic acid at elevated temperature and pressure. These are not ideal process conditions, particularly as phosphotungstic acid is a significant safety and environmental hazard, and the tertiary alcohol is required in a substantial excess. The tertiary alcohol is quite often the more expensive reagent when producing tertiary alkyl organic peroxides.

US3308163 discloses a process for preparing organic peroxides by a condensation reaction between an organic alcohol and an organic hydroperoxide in the presence of an acidic solid ion exchange resin. Azeotropic removal of water during the condensation reaction is shown to be essential for that process.

US3919326 discloses a process for preparing organic peroxides by a condensation reaction between a poly(hydroxyisopropyl)aryl compound containing at least two alpha-hydroxyisopropyl groups with a chemically equivalent quantity of t-alkyl hydroperoxide in the presence of p-toluene sulfonic acid and a large volume of organic solvents. The process required azeotropic removal of water during the condensation reaction, and even at an increased process temperature of 70-80°C the reaction was still relatively slow (>3.5h to completion).

EP0967194 discloses a process for preparing dicumylperoxide by a condensation reaction between cumyl alcohol and cumene hydroperoxide in the presence of an aromatic sulfonic acid. The process disclosed therein resulted in unimpressive product yields (<80%).

US2016207882 discloses a process for preparing tertiary alkyl organic peroxides by a condensation reaction between a tertiary organic alcohol and a tertiary organic hydroperoxide in the presence of a sulfonic acid and sulfuric acid, wherein the mole ratio of sulfonic acid to tertiary alcohol is between 0.1 and 0.6. The worked examples of US2016207882 show that a substantial amount of sulfuric acid was required (>60 mol% relative to the molar amount of organic alcohol), which may explain why the process required numerous aqueous washing steps of the final organic layer (presumably to remove the substantial amounts of acid waste salts) and did not consistently generate good product yields.

There remains a need for a process for preparing a tertiary alkyl organic peroxide that addresses all the technical issues set out above.

### Description of the invention

The inventors have now developed a highly effective process for producing tertiary alkyl organic peroxides that address all the above technical issues.

In that respect, in a first aspect the present invention relates to a process for preparing a tertiary alkyl organic peroxide, the process comprising:
a) in a first condensation stage, reacting a component containing at least one tertiary alcohol group with a compound containing at least one tertiary hydroperoxide function, in the presence of a catalyst and optional co-catalyst, to form a mixture comprising an organic phase comprising the tertiary alkyl organic peroxide and an aqueous phase,
b) in a dewatering stage, separating the aqueous phase from the organic phase, and
c) in a second condensation stage, continuing reacting the component containing at least one tertiary alcohol group with the compound containing at least one tertiary hydroperoxide group, in the presence of a catalyst and optional co-catalyst,

wherein, in the first condensation stage, the mole ratio between the compound containing at least one tertiary hydroperoxide function and the component containing at least one tertiary alcohol group is at least 3:1, and
wherein no dewatering takes place during the first condensation stage.

An unexpected finding was that introducing an intermediate dewatering step in the reaction removed the need for azeotropic dewatering during the first condensation stage, allowed for increased reaction temperatures (thus reducing the overall reaction time), and significantly reduced the overall amount of catalyst required (thereby substantially reducing the amount of salt wastes produced), all without sacrificing product yield (>90%) and maintaining very high selectivity towards the desired product. To reliably obtain these advantages, however, it was found that the molar ratio between the compound containing at least one tertiary hydroperoxide function and the component containing at least one tertiary alcohol group had to be at least 3:1. That ratio was found to be critical to the success of the process: below that 3:1 ratio, the resulting suspension was highly viscous making it very difficult to stir, and the mixture contained undissolved tertiary alcohol which resulted in fouling of the reactor walls and diminished the reaction kinetics, i.e., led to long reaction times. It was also found that the selectivity of the reaction could be improved by increasing that mole ratio. As such, it is preferred if the mole ratio between the compound containing at least one tertiary hydroperoxide function and the component containing at least one tertiary alcohol group is greater than 3:1, preferably at least 3.5:1, more preferably at least 4:1.

For the avoidance of doubt, it should be understood that in the intermediate dewatering step b) the organic phase and aqueous phase of step a) are allowed to settle/separate (generally by stopping agitation/stirring), after which the separated aqueous layer is removed.

Preferably, the component containing at least one tertiary alcohol group and/or the compound containing at least one tertiary hydroperoxide function comprise one or more aromatic functions such that the tertiary alkyl organic peroxide comprises at least one aromatic function.

Preferably, the component containing at least one tertiary alcohol group is selected from α,α'-dihydroxy-1,3-diisopropylbenzene, α,α'-dihydroxy-1,4-diisopropylbenzene, or mixtures thereof.

Preferably, the compound containing at least one tertiary hydroperoxide group is selected from tert-butyl hydroperoxide, tert-amyl hydroperoxide, cumyl hydroperoxide, 1,1,3,3-tetramethylbutyl hydroperoxide, isopropylcumyl hydroperoxide, pinane hydroperoxide (2,6,6-trimethylbicyclo[3.1.1]heptyl hydroperoxide), para-menthane hydroperoxide, and mixtures thereof. tert-Butyl hydroperoxide is most preferred.

Preferably, the catalyst is an acid catalyst, preferably selected from aliphatic sulfonic acids, aromatic sulfonic acids, and/or perchloric acid. Preferred sulfonic acids include, but are not limited to, para-toluenesulfonic acid, methanesulfonic acid, trifluoromethanesulfonic acid, benzenesulfonic acid, 1,5-naphthalenedisulfonic acid, ethanesulfonic acid, and mixtures thereof. Para-toluenesulfonic acid (PTSA) is most preferred.

The process may employ a co-catalyst in the first and/or second condensation stage. Preferred co-catalysts include, but are not limited to, at least one water soluble salt of an inorganic acid. Preferred water soluble inorganic acid salts include, but are not limited to, perchlorate salts, sulfate salts, perborate salts, and mixtures thereof. Preferably, the cationic counterion of the inorganic acid salts is an alkali(ne) metal cation, preferably sodium, potassium, magnesium or calcium. More preferred co-catalysts include sodium perchlorate, potassium sulfate, potassium perchlorate, magnesium sulfate, sodium perchlorate, magnesium sulfate, and mixtures thereof. Sodium perchlorate is most preferred.

Thus, in a preferred embodiment, the process for preparing a tertiary alkyl organic peroxide comprises:
a) in a first condensation stage, reacting α,α'-dihydroxy-1,3-diisopropylbenzene, α,α'-dihydroxy-1,4-diisopropylbenzene, or mixtures thereof, with tert-butyl hydroperoxide, in the presence of a catalyst (preferably para-toluenesulfonic acid) and optional co-catalyst (preferably sodium perchlorate), to form a mixture comprising an organic phase comprising the tertiary alkyl organic peroxide and an aqueous phase,
b) in a dewatering stage, separating the aqueous phase from the organic phase, and
c) in a second condensation stage, continuing reacting α,α'-dihydroxy-1,3-diisopropylbenzene, α,α'-dihydroxy-1,4-diisopropylbenzene, or mixtures thereof, with tert-butyl hydroperoxide, in the presence of a catalyst (preferably para-toluenesulfonic acid) and optional co-catalyst (preferably sodium perchlorate), wherein, in the first condensation stage, the mole ratio between tert-butyl hydroperoxide and α,α'-dihydroxy-1,3-diisopropylbenzene, α,α'-dihydroxy-1,4-diisopropylbenzene, or mixtures thereof, is at least 3:1, and wherein no dewatering takes place during the first condensation stage.

Preferably, a first portion of catalyst and optional co-catalyst is added in the first condensation stage and a second portion of catalyst and optional co-catalyst is added in the second condensation stage. Preferably, the first portion of catalyst is added in an amount of from 3-15 mol %, preferably 4-10 mol % (relative to the initial number of moles of the component containing at least one tertiary alcohol group) in the first condensation stage and the second portion of catalyst is added in an amount of from 3-15 mol %, preferably 4-10 mol % (relative to the initial number of moles of the component containing at least one tertiary alcohol group) in the second condensation stage. If a co-catalyst is used, then it is preferable if a first portion of co-catalyst is added in an amount of from 2-10 mol %, preferably 3-8 mol % (relative to the initial number of moles of the component containing at least one tertiary alcohol group) in the first condensation stage and a second portion of co-catalyst is added in an amount of from 2-10 mol %, preferably 3-8 mol % (relative to the initial number of moles of the component containing at least one tertiary alcohol group) in the second condensation stage. It should be understood that the first portion of catalyst/optional co-catalyst does not need to be the same amount as the second portion of catalyst/optional co-catalyst.

Thus, in another preferred embodiment, the process for preparing a tertiary alkyl organic peroxide comprises:
a) in a first condensation stage, reacting a component containing at least one tertiary alcohol group with a compound containing at least one tertiary hydroperoxide function, in the presence of 3-15 mol% catalyst (relative to the initial number of moles of the component containing at least one tertiary alcohol group) and optionally 2-10 mol% co-catalyst (relative to the initial number of moles of the component containing at least one tertiary alcohol group), to form a mixture comprising an organic phase comprising the tertiary alkyl organic peroxide and an aqueous phase,
b) in a dewatering stage, separating the aqueous phase from the organic phase, and
c) in a second condensation stage, continuing reacting the component containing at least one tertiary alcohol group with the compound containing at least one tertiary hydroperoxide group, in the presence of 3-15 mol% catalyst (relative to the initial number of moles of the component containing at least one tertiary alcohol group) and optionally 2-10 mol% co-catalyst (relative to the initial number of moles of the component containing at least one tertiary alcohol group),

wherein, in the first condensation stage, the mole ratio between the compound containing at least one tertiary hydroperoxide function and the component containing at least one tertiary alcohol group is at least 3:1, and
wherein no dewatering takes place during the first condensation stage.

In a more preferred embodiment, the process for preparing a tertiary alkyl organic peroxide comprises:
a) in a first condensation stage, reacting α,α'-dihydroxy-1,3-diisopropylbenzene, α,α'-dihydroxy-1,4-diisopropylbenzene, or mixtures thereof, with tert-butyl hydroperoxide, in the presence of 3-15 mol% catalyst (preferably para-toluenesulfonic acid; mol% relative to the initial number of moles of the component containing at least one tertiary alcohol group) and optionally 2-10 mol% co-catalyst (preferably sodium perchlorate; mol% relative to the initial number of moles of the component containing at least one tertiary alcohol group), to form a mixture comprising an organic phase comprising the tertiary alkyl organic peroxide and an aqueous phase,
b) in a dewatering stage, separating the aqueous phase from the organic phase, and
c) in a second condensation stage, continuing reacting α,α'-dihydroxy-1,3-diisopropylbenzene, α,α'-dihydroxy-1,4-diisopropylbenzene, or mixtures thereof, with tert-butyl hydroperoxide, in the presence of 3-15 mol% catalyst (preferably para-toluenesulfonic acid; mol% relative to the initial number of moles of the component containing at least one tertiary alcohol group) and optionally 2-10 mol% co-catalyst (preferably sodium perchlorate; mol% relative to the initial number of moles of the component containing at least one tertiary alcohol group), the mole ratio between tert-butyl hydroperoxide and α,α'-dihydroxy-1,3-diisopropylbenzene, α,α'-dihydroxy-1,4-diisopropylbenzene, or mixtures thereof, is at least 3:1, and wherein no dewatering takes place during the first condensation stage.

As noted above, the process conditions allow for a higher reaction temperature. It has been found that the optimal process temperature in the first and/or second condensation stage is from 55 to 80°C, preferably from 62°C to 80°C, preferably from 65 °C to 80 °C, preferably of from 67 °C to 80 °C, and more preferably of from 69 °C to 80 °C. The dewatering stage b) may be performed at a lower temperature than the first and/or second condensation stages (e.g., at about 50-60°C).

It has also been found that whilst organic solvents may be used in the process disclosed herein, they are not essential to the success of the process disclosed herein. As such, the reaction of the first and/or second condensation stage may be carried out in the absence of an organic solvent. This is beneficial in terms of process cost and safety, and substantially improves the environmental profile of the process.

As noted above, it is essential that the molar ratio between the compound containing at least one tertiary hydroperoxide function and the component containing at least one tertiary alcohol group must be at least 3:1 (i.e., the hydroperoxide is present in excess). As such, to improve the overall efficiency of the process, it is preferred if the process further comprises recycling at least part of the compound containing at least one tertiary hydroperoxide group present in the product of the second condensation stage to the first condensation stage.

Thus, in another preferred embodiment, the process for preparing a tertiary alkyl organic peroxide comprises:
a) in a first condensation stage, reacting a component containing at least one tertiary alcohol group with a compound containing at least one tertiary hydroperoxide function, in the presence of a catalyst and optional co-catalyst, to form a mixture comprising an organic phase comprising the tertiary alkyl organic peroxide and an aqueous phase,
b) in a dewatering stage, separating the aqueous phase from the organic phase, and
c) in a second condensation stage, continuing reacting the component containing at least one tertiary alcohol group with the compound containing at least one tertiary hydroperoxide group, in the presence of a catalyst,

wherein, in the first condensation stage, the mole ratio between the compound containing at least one tertiary hydroperoxide function and the component containing at least one tertiary alcohol group is at least 3:1,
wherein no dewatering takes place during the first condensation stage, and
wherein at least part of the compound containing at least one tertiary hydroperoxide group present in the product of the second condensation stage is recycled to the first condensation stage.

In a more preferred embodiment, the process for preparing a tertiary alkyl organic peroxide comprises:
a) in a first condensation stage, reacting α,α'-dihydroxy-1,3-diisopropylbenzene, α,α'-dihydroxy-1,4-diisopropylbenzene, or mixtures thereof, with tert-butyl hydroperoxide, in the presence of 3-15 mol% catalyst (preferably para-toluenesulfonic acid; mol% relative to the initial number of moles of the component containing at least one tertiary alcohol group) and optionally 2-10 mol% co-catalyst (preferably sodium perchlorate; mol% relative to the initial number of moles of the component containing at least one tertiary alcohol group), to form a mixture comprising an organic phase comprising the tertiary alkyl organic peroxide and an aqueous phase,
b) in a dewatering stage, separating the aqueous phase from the organic phase, and
c) in a second condensation stage, continuing reacting α,α'-dihydroxy-1,3-diisopropylbenzene, α,α'-dihydroxy-1,4-diisopropylbenzene, or mixtures thereof, with tert-butyl hydroperoxide, in the presence of 3-15 mol% catalyst (preferably para-toluenesulfonic acid; mol% relative to the initial number of moles of the component containing at least one tertiary alcohol group) and optionally 2-10 mol% co-catalyst (preferably sodium perchlorate; mol% relative to the initial number of moles of the component containing at least one tertiary alcohol group), the mole ratio between tert-butyl hydroperoxide and α,α'-dihydroxy-1,3-diisopropylbenzene, α,α'-dihydroxy-1,4-diisopropylbenzene, or mixtures thereof, is at least 3:1,

wherein no dewatering takes place during the first condensation stage, and
wherein at least part of the tert-butyl hydroperoxide present in the product of the second condensation stage is recycled to the first condensation stage.

The process disclosed herein may be performed as a batchwise process or may be performed in a continuous manner.

It is noted that various elements of the present invention, including but not limited to preferred ranges for the various parameters, can be combined unless they are mutually exclusive.

The invention will be elucidated by the following examples without being limited thereto or thereby.

### Examples

### Example 1 (Mol. Ratio t-OOH : t-OH = 4.1 :1)

A glass reactor of 1L equipped with a bottom valve, mechanical stirrer, thermometer and a reflux condenser was charged with 342.9g (2.66 mol) TBHP as a 70 wt% solution in water. After charging 126.2g (0.65 mol) α,α'-dihydroxy-1,3-diisopropylbenzene and 6.63g (0.032 mol) of a 60 wt% aqueous solution of sodium perchlorate the mixture was heated to 62.5 °C. Within 5 minutes 9.51g (0.036 mol) of a 65 wt% aqueous solution of *p*-toluene sulphonic acid was added to the mixture. After 20 min the temperature was lowered to 60 °C. Stirring was stopped and the aqueous phase was removed via the bottom valve.

Stirring was started and 6.63g (0.032 mol) of a 60 wt% aqueous solution of sodium perchlorate was added and the mixture was heated to 62.5 °C. Within 5 minutes 9.51g (0.036 mol) of a 65 wt% aqueous solution of *p*-toluene sulphonic acid was added to the mixture. The mixture was cooled to 60 °C after stirring for 100 min. Stirring was stopped and the aqueous phase was removed via the bottom valve.

Amount of catalyst (PTSA) added (relative to the initial number of moles of the component containing at least one tertiary alcohol group): 5.5 mol% in first condensation stage ((0.036/0.65)*100); 5.5 mol% in second condensation stage; 11 mol% in total.

Amount of co-catalyst (NaClO₄) added (relative to the initial number of moles of the component containing at least one tertiary alcohol group): 5 mol% in first condensation stage ((0.032/0.60)*100); 5 mol% in second condensation stage; 10 mol% in total.

GC analysis of the organic phase, excluding TBHP, showed the following composition: 95.53% α,α'-bis-(*tert*-butylperoxy)-1,3-diisopropylbenzene, 0.76% α-(*tert-*butylperoxy)-α'-hydroxy-1,3-diisopropylbenzene and 2.53% α-(*tert*-butylperoxy)isopropyl-3-isopropenylbenzene.

### Example 2 (Mol. Ratio t-OOH : t-OH = 4.1 :1)

A glass reactor of 1L equipped with a bottom valve, mechanical stirrer, thermometer and a reflux condenser was charged with 171.7g TBHP as a 70 wt% solution in water. After charging 63.2g α,α'-dihydroxy-1,3-diisopropylbenzene and 3.32g of a 60 wt% aqueous solution of sodium perchlorate the mixture was heated to 62.5 °C. Within 5 minutes 4.76g of a 65 wt% aqueous solution of *p*-toluene sulphonic acid was added to the mixture. After 20 min the temperature was lowered to 60 °C. Stirring was stopped and the aqueous phase was removed via the bottom valve.

Stirring was started and 3.32g of a 60 wt% aqueous solution of sodium perchlorate was added and the mixture was heated to 62.5 °C. Within 5 minutes 9.52g of a 65 wt% aqueous solution of *p*-toluene sulphonic acid was added to the mixture. The mixture was cooled to 60 °C after stirring for 80 min. Stirring was stopped and the aqueous phase was removed via the bottom valve.

Amount of catalyst (PTSA) added (relative to the initial number of moles of the component containing at least one tertiary alcohol group): 5 mol% in first condensation stage; 10 mol% in second condensation stage; 15 mol% in total.

Amount of co-catalyst (NaClO₄) added (relative to the initial number of moles of the component containing at least one tertiary alcohol group): 5 mol% in first condensation stage; 5 mol% in second condensation stage; 10 mol% in total.

GC analysis of the organic phase, excluding TBHP, showed the following composition: 95.91% α,α'-bis-(*tert*-butylperoxy)-1,3-diisopropylbenzene, 0.50% α-(*tert-*butylperoxy)-α'-hydroxy-1,3-diisopropylbenzene and 2.51% α-(*tert*-butylperoxy)isopropyl-3-isopropenylbenzene.

### Example 3 (Mol. Ratio t-OOH : t-OH = 4.1 :1)

A glass reactor of 1L equipped with a bottom valve, mechanical stirrer, thermometer, and a reflux condenser was charged with 172.26g TBHP as a 70 wt% solution in water. After charging 63.4g α,α'-dihydroxy-1,3-diisopropylbenzene and 3.33g of a 60 wt% aqueous solution of sodium perchlorate the mixture was heated to 67.5 °C. Within 5 minutes 4.78g of a 65 wt% aqueous solution of *p*-toluene sulphonic acid was added to the mixture. After 20 min the temperature was lowered to 60 °C. Stirring was stopped and the aqueous phase was removed via the bottom valve.

Stirring was started and 3.33g of a 60 wt% aqueous solution of sodium perchlorate was added and the mixture was heated to 67.5 °C. Within 5 minutes 5.73g of a 65 wt% aqueous solution of *p*-toluene sulphonic acid was added to the mixture. The mixture was cooled to 60 °C after stirring for 20 min. Stirring was stopped and the aqueous phase was removed via the bottom valve.

Amount of catalyst (PTSA) added (relative to the initial number of moles of the component containing at least one tertiary alcohol group): 6 mol% in first condensation stage; 7 mol% in second condensation stage; 13 mol% in total.

Amount of co-catalyst (NaClO₄) added (relative to the initial number of moles of the component containing at least one tertiary alcohol group): 5 mol% in first condensation stage; 5 mol% in second condensation stage; 10 mol% in total.

GC analysis of the organic phase, excluding TBHP, showed the following composition: 95.22% α,α'-bis-(*tert*-butylperoxy)-1,3-diisopropylbenzene, 0.39% α-(*tert-*butylperoxy)-α'-hydroxy-1,3-diisopropylbenzene and 2.17% α-(*tert*-butylperoxy)isopropyl-3-isopropenylbenzene.

### Example 4 (Mol. Ratio t-OOH : t-OH = 3 :1)

A glass reactor of 1L equipped with a bottom valve, mechanical stirrer, thermometer, and a reflux condenser was charged with 163.09g TBHP as a 70 wt% solution in water. After charging 80.0g α,α'-dihydroxy-1,3-diisopropylbenzene and 4.21g of a 60 wt% aqueous solution of sodium perchlorate the mixture was heated to 67.5 °C. Within 5 minutes 4.72g of a 65 wt% aqueous solution of *p*-toluene sulphonic acid was added to the mixture. After 60 min the temperature was lowered to 60 °C. Stirring was stopped and the aqueous phase was removed via the bottom valve.

Stirring was started and 4.21g of a 60 wt% aqueous solution of sodium perchlorate was added and the mixture was heated to 67.5 °C. Within 5 minutes 4.72g of a 65 wt% aqueous solution of *p*-toluene sulphonic acid was added to the mixture. The mixture was cooled to 60 °C after stirring for 240 min. Stirring was stopped and the aqueous phase was removed via the bottom valve.

Amount of catalyst (PTSA) added (relative to the initial number of moles of the component containing at least one tertiary alcohol group): 4.3 mol% in first condensation stage; 4.3 mol% in second condensation stage; 8.6 mol% in total.

Amount of co-catalyst (NaClO₄) added (relative to the initial number of moles of the component containing at least one tertiary alcohol group): 5 mol% in first condensation stage; 5 mol% in second condensation stage; 10 mol% in total.

GC analysis of the organic phase, excluding TBHP, showed the following composition: 92.25% α,α'-bis-(*tert*-butylperoxy)-1,3-diisopropylbenzene, 2.17% α-(*tert-*butylperoxy)-α'-hydroxy-1,3-diisopropylbenzene and 4.01% α-(*tert*-butylperoxy)isopropyl-3-isopropenylbenzene.

### Example 5 (Mol. Ratio t-OOH : t-OH = 4.1 :1)

A glass reactor of 1L equipped with a bottom valve, mechanical stirrer, thermometer, and a reflux condenser was charged with 217.36g TBHP as a 70 wt% solution in water. After charging 80.0g α,α'-dihydroxy-1,3-diisopropylbenzene and 4.20g of a 60 wt% aqueous solution of sodium perchlorate the mixture was heated to 69.5 °C. Within 5 minutes 4.72g of a 65 wt% aqueous solution of *p*-toluene sulphonic acid was added to the mixture. After 20 min the temperature was lowered to 60 °C. Stirring was stopped and the aqueous phase was removed via the bottom valve.

Stirring was started and 4.20g of a 60 wt% aqueous solution of sodium perchlorate was added and the mixture was heated to 69.5 °C. Within 5 minutes 4.72g of a 65 wt% aqueous solution of *p*-toluene sulphonic acid was added to the mixture. The mixture was cooled to 60 °C after stirring for 60 min. Stirring was stopped and the aqueous phase was removed via the bottom valve.

Amount of catalyst (PTSA) added (relative to the initial number of moles of the component containing at least one tertiary alcohol group): 4.3 mol% in first condensation stage; 4.3 mol% in second condensation stage; 8.6 mol% in total.

Amount of co-catalyst (NaClO₄) added (relative to the initial number of moles of the component containing at least one tertiary alcohol group): 5 mol% in first condensation stage; 5 mol% in second condensation stage; 10 mol% in total.

GC analysis of the organic phase, excluding TBHP, showed the following composition: 94.72% α,α'-bis-(*tert*-butylperoxy)-1,3-diisopropylbenzene, 0.83% α-(*tert-*butylperoxy)-α'-hydroxy-1,3-diisopropylbenzene and 3.04% α-(*tert*-butylperoxy)isopropyl-3-isopropenylbenzene.

### Example 6 (Mol. Ratio t-OOH : t-OH = 4.1 :1)

A glass reactor of 1L equipped with a bottom valve, mechanical stirrer, thermometer, and a reflux condenser was charged with 244.5g TBHP as a 70 wt% solution in water. After charging 90.0g α,α'-dihydroxy-1,3-diisopropylbenzene and 2.70g of solid sodium perchlorate the mixture was heated to 69.5 °C. Within 5 minutes 5.31g of a 65 wt% aqueous solution of *p*-toluene sulphonic acid was added to the mixture. After 20 min the temperature was lowered to 60 °C. Stirring was stopped and the aqueous phase was removed via the bottom valve.

Stirring was started and 2.70g of solid sodium perchlorate was added and the mixture was heated to 69.5 °C. Within 5 minutes 5.31g of a 65 wt% aqueous solution of *p-*toluene sulphonic acid was added to the mixture. The mixture was cooled to 60 °C after stirring for 50 min. Stirring was stopped and the aqueous phase was removed via the bottom valve.

Amount of catalyst (PTSA) added (relative to the initial number of moles of the component containing at least one tertiary alcohol group): 4.3 mol% in first condensation stage; 4.3 mol% in second condensation stage; 8.6 mol% in total.

Amount of co-catalyst (NaClO₄) added (relative to the initial number of moles of the component containing at least one tertiary alcohol group): 5 mol% in first condensation stage; 5 mol% in second condensation stage; 10 mol% in total.

GC analysis of the organic phase, excluding TBHP, showed the following composition: 94.82% α,α'-bis-(*tert*-butylperoxy)-1,3-diisopropylbenzene, 1.41% α-(*tert-*butylperoxy)-α'-hydroxy-1,3-diisopropylbenzene and 2.52% α-(*tert*-butylperoxy)isopropyl-3-isopropenylbenzene.
In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than to mean 'consisting of'. All prior teachings acknowledged above are hereby incorporated by reference. No acknowledgement of any prior published document herein should be taken to be an admission or representation that the teaching thereof was common general knowledge in Europe or elsewhere at the date hereof.

## Claims

1. A process for preparing a tertiary alkyl organic peroxide, the process comprising:
a) in a first condensation stage, reacting a component containing at least one tertiary alcohol group with a compound containing at least one tertiary hydroperoxide function, in the presence of a catalyst and optional co-catalyst, to form a mixture comprising an organic phase comprising the tertiary alkyl organic peroxide and an aqueous phase,
b) in a dewatering stage, separating the aqueous phase from the organic phase, and
c) in a second condensation stage, continuing reacting the component containing at least one tertiary alcohol group with the compound containing at least one tertiary hydroperoxide group, in the presence of a catalyst and optional co-catalyst,
wherein, in the first condensation stage, the mole ratio between the compound containing at least one tertiary hydroperoxide function and the component containing at least one tertiary alcohol group is at least 3:1, and
wherein no dewatering takes place during the first condensation stage.

2. A process as claimed in claim 1, wherein the component and/or the compound comprises one or more aromatic functions such that the tertiary alkyl organic peroxide comprises at least one aromatic function.

3. A process as claimed in claim 1 or claim 2, wherein the component containing at least one tertiary alcohol group is selected from α,α'-dihydroxy-1,3-diisopropylbenzene, α,α'-dihydroxy-1,4-diisopropylbenzene, or mixtures thereof.

4. A process as claimed in any preceding claim, wherein the compound containing at least one tertiary hydroperoxide group is selected from tert-butyl hydroperoxide, tert-amyl hydroperoxide, cumyl hydroperoxide, 1,1,3,3-tetramethylbutyl hydroperoxide, isopropylcumyl hydroperoxide, pinane hydroperoxide (2,6,6-trimethylbicyclo[3.1.1]heptyl hydroperoxide), para-menthane hydroperoxide, or mixtures thereof.

5. A process as claimed in any preceding claim, wherein the reaction of the first and/or second condensation stage is carried out in the absence of organic solvent.

6. A process as claimed in any preceding claim, wherein the catalyst is an acid catalyst, preferably selected from aliphatic sulfonic acids, aromatic sulfonic acids, and/or perchloric acid.

7. A process as claimed in any preceding claim, wherein the catalyst is added in the first and/or second condensation stage in an amount of from 3 mol% to 15 mol% relative to the initial number of moles of the component containing at least one tertiary alcohol group.

8. A process as claimed in claim 7, wherein a first portion of the catalyst is added in the first condensation stage in an amount of from 3 mol% to 15 mol% relative to the initial number of moles of the component containing at least one tertiary alcohol group, and wherein a second portion of the catalyst is added in the second condensation stage in an amount of from 3 mol% to 15 mol% relative to the initial number of moles of the component containing at least one tertiary alcohol group.

9. A process as claimed in any preceding claim, wherein the first and second condensation stage are performed at a temperature from 55 °C to 80°C, preferably from 62°C to 80°C, preferably from 65 °C to 80 °C, preferably of from 67 °C to 80 °C, more preferably of from 69 °C to 80 °C.

10. A process as claimed in any preceding claim, wherein the reaction of the first and/or second condensation stage is carried out in the presence of a co-catalyst.

11. A process as claimed in claim 10, wherein the co-catalyst is added in the first and/or second condensation stage in an amount of from 2 mol% to 10 mol% relative to the initial number of moles of the component containing at least one tertiary alcohol group.

12. A process as claimed in claim 11, wherein a first portion of the co-catalyst is added in the first condensation stage in an amount of from 2 mol% to 10 mol% relative to the initial number of moles of the component containing at least one tertiary alcohol group, and wherein a second portion of the co-catalyst is added in the second condensation stage in an amount of from 2 mol% to 10 mol% relative to the initial number of moles of the component containing at least one tertiary alcohol group.

13. A process as claimed in any preceding claim, wherein the catalyst is para-toluene sulfonic acid and the co-catalyst is sodium perchlorate.

14. A process as claimed in any preceding claim, wherein the component containing at least one tertiary alcohol group is selected from α,α'-dihydroxy-1,3-diisopropylbenzene, α,α'-dihydroxy-1,4-diisopropylbenzene, or mixtures thereof, and wherein the compound containing at least one tertiary hydroperoxide group is tert-butyl hydroperoxide.

15. A process as claimed in any preceding claim, further comprising recycling at least part of the compound containing at least one tertiary hydroperoxide group present in the product of the second condensation stage to the first condensation stage.
